# EUROPEAN PATENT SPECIFICATION

(11) **EP 1 397 179 B1**
(45) Date of publication and mention of the grant of the patent: **15.10.2008**
(21) Application number: 02720740.6
(22) Date of filing: 12.04.2002
(51) Int. Cl.: A61N 1/365

(54) **HEART STIMULATOR**
HERZSCHRITTMACHER
STIMULATEUR CARDIAQUE

(30) Priority: 31.05.2001 SE 0101918
(43) Date of publication of application: 17.03.2004
(73) Proprietor: St. Jude Medical AB, 175 84 Järfälla (SE)
(72) Inventor: STRANDBERG, Hans, S-172 36 Sundbyberg (SE)
(86) International application number: PCT/SE2002/000738
(87) International publication number: WO 2002/096513

(56) References cited:
- EP-A1- 0 634 192
- WO-A1-82/00581
- WO-A1-95/19806
- DE-A1- 19 927 615
- US-A- 5 409 009
- US-A- 5 602 342
- US-A- 5 995 870
- US-A- 6 115 633
- US-A1- 2001 005 790
- US-A1- 2001 021 864

## Description

### Technical field

The present invention relates to a heart stimulator for multi-site stimulation of a patient's heart comprising a pulse generator and a measuring device for measuring a physiological cardiac parameter and delivering a corresponding output signal to a control unit for controlling time intervals between pulses delivered to different stimulation sites in the heart depending on said output signal according to a predetermined criterion.

### Background

At multi-site stimulation there are different methods known of tuning time intervals between pulses delivered to different stimulation sites in the human heart. Thus it is well known to control the right side atrium to ventricular delay (AV-delay) by using external ultrasound technique to measure the cardiac output or by measuring work condition of the patient. These previously known methods are intended for temporary use and are related to the pumping efficiency of the heart and to the relation between the AV-delay and stimulation rate.

Multi-site cardiac stimulators are also described in e.g. US 5,995,870 and US 6,181,968. US 5,995,870 discloses a multi-site cardiac stimulator with a contraction detection sensor of at least one of the ventricles to detect an instant of a beginning of valvular opening to determine an optimal electrode configuration operation from various possible configurations. US 6,181,968 describes an implantable medical device of multi-site type having electrodes in at least two neighbouring cardiac sites, e.g. right and left ventricular sites, and/or right and left atrial sites. A sensing circuit is provided to detect depolarisation on corresponding sites to indicate loss of capture and allow for setting the stimulation amplitude above the capture threshold.

US 5 409 009 describes a blood flow sensor for use to control the operation of an implantable medical device, like a pacemaker, and DE 199 27 615 discloses a detector for determining the blood supply to myocardium to sense an ischaemic state. When an incipient ischaemic state is then sensed a pacemaker is controlled from the detector to emit stimulation pulses such that the development of the ischaemia is limited.

It has appeared that heart conditions depend very much on blood perfusion of the heart itself and in worse condition ischemic situations can be created by unsuitable cardiac contraction sequences. Already small changes in the stimulation sequence seriously influence the blood flow in the heart's own vessels.

The purpose of the present invention is to control this perfusion blood flow to be in a way optimal and avoid unsuitable blood flow conditions for the heart. A device according to the first past of claim 1 is known from US-A-6 115 633.

### Disclosure of the Invention

This purpose is obtained by a heart stimulator of the kind defined in the introductory portion of the description and having the characterizing features of claim 1.

Thus with the present invention a pacemaker is provided wherein the measuring device comprises a ring-shaped electrode sized to fit within a blood vessel for the perfusion blood flow of the patient's heart, a counter electrode intended to be brought into contact with the blood, and a measuring unit adapted to measure the voltage or current between said electrodes to determine therefrom the perfusion blood flow in said vessel as said physiological parameter. Such an electrochemical flow measuring device, which is described in greater detail in Swedish patent application No. 0101917-3 filed simultaneously with the present application, cf. also US 5,602,342, makes continuous measurements of the blood flow possible in a simple and reliable way. Only comparison of blood flow values for different stimulation interval situations, i.e. increases and decreases in the flow under different conditions, is needed and no measurement of absolute flow values. Further, the timing of the therapeutic stimulation pulses at multi-site stimulation of the heart is controlled depending on the measured perfusion blood flow in the heart itself. The therapy can consequently be tuned to give the heart itself the best working condition by increasing, when necessary, the blood flow in the hearts own vessels. This control of the timing of the stimulation therapy in response to the measured perfusion blood flow can be used continuously, also when the patient is out from the hospital. By "multi-site" is meant in this connection at least two of the four different cavities of the heart, and/or also the possibility of more than one stimulation site in one cavity. Thus the new therapy can be used e.g. for controlling the ordinary AV-delay, and it can be used for instance in connection with biventricular stimulation, i.e. when both the left and right ventricles are stimulated.

In the heart stimulator according to the invention the ring-shaped electrode is sized to fit within and is adapted for measuring the blood flow in coronary sinus vein. A suitable site for measuring the blood flow is somewhere in the hearts own venous system and preferably in and near the orifice of the coronary sinus vein.

According to another advantageous embodiments of the heart stimulator according to the invention the measuring device is adapted to measure changes in the perfusion blood flow in response to changes of the time intervals. The control unit suitably comprises a memory for storage of latest measured flow values and associated time interval values. The memory can preferably be adapted to store measured flow values from at least two consecutive heart cycles with constant parameter settings, and the control unit can be adapted to compare measured flow values with stored flow values for constant stimulation rate and adjust said time intervals depending on the result of the comparison. Thus the comparison of the flow values is preferably done between stored values and measured values for constant heartbeat rates but for different delay intervals. The delay parameter shall be adjusted in that direction which gives the highest blood flow. Some heartbeats later new flow values from last new heartbeats are stored and can be compared to the previous ones. If the new result is an increased flow further adjustment of the next beat is done in the same direction as before, but if the result is a decreased flow a return to the last parameter setting is done.

According to yet another advantageous embodiment of the heart stimulator according to the invention the control unit is adapted to adjust one of said time intervals at a time. This is the simplest way of adjustment where one parameter at each time is adjusted until an optimal setting is achieved, whereafter another parameter is adjusted and so on. The four parameters of primary interest are a) delay time between right and left ventricular at stimulation in each one of these cavities, b) delay time between right and left ventricular when there is a spontaneous heartbeat starting before in one of the chambers, delay time between atrial and ventricular activation c) when both activations are stimulated d) when atrial activation is spontaneous. Additional interval parameters are of interest if left atrium is also involved in sensing and stimulation.

According to another advantageous embodiment of the heart stimulator according to the invention the control unit is adapted for multi-interval adjustment. Thus two or more interval parameters can be adjusted at the same time. Such a multiparameter tuning will result in faster reaching of the best settings.

According to still another advantageous embodiment of the heart stimulator according to the invention said criterion means that the minimum blood flow during a cardiac cycle is increased as much as possible. This results in a minimum flow pulsation in the vein system which normally is of high interest from clinical aspect. Thus, the blood flow of the heart is supplied from the aorta near the aortic valves, the blood pressure there being strongly pulsating during a heartbeat. From the broad coronary arteries through the smallest vessels to the coronary veins the blood flow is mechanically low pass filtered and becomes more constant and steady. This is a situation if the heart is working correctly. However, if the contraction sequence of the heart is in disorder with the pulsating aortic blood flow, the perfusion flow is not promoted by the contraction in different parts of the heart. On the contrary the blood flow can temporarily be stopped and even reversed. By checking the minimum blood flow during one heartbeat a very good indication of non-suitable timing of the contraction sequence is obtained.

According to another advantageous embodiment of the heart stimulator according to the invention said determining means comprises and external determining apparatus. Then very precise and accurate flow measurements can be performed. It will be possible to scan all parameter settings with different heart rates and all corresponding flow values can stored. After compilation of these data the implanted device can be programmed with a set of suitable parameters. In practice this means that a table of data is transferred into a memory of the implanted device. The implanted device then needs no flow measuring means but only means for measuring time intervals for spontaneous and stimulated heart rates. Delay parameter values related to these heart rates are then obtained from the table which have been stored from the external equipment.

### Brief description of the drawings

To explain the invention in greater detail embodiments of the heart stimulator according to the invention will now be described with reference to the enclosed drawings, on which
- Figure 1: shows schematically an embodiment of an implanted heart stimulator for multi-site stimulation according to the invention,
- Figure 2: is a block diagram of the electronic circuitry of an embodiment of the heart stimulator according to the invention, and
- Figure 3: illustrates an alternative embodiment of the heart stimulator according to the invention.

### Description of preferred embodiments

The heart stimulator 2, like a pacemaker or a defibrillator, includes a measuring device for measuring the perfusion blood flow of a patient's heart 6. This measuring device is preferably of an electrolytic type comprising a ring-shaped measuring electrode 4 intended for implantation in the coronary sinus vein 8 and a suitable counter electrode, e.g. one of the stimulation electrodes, the blood flow in the coronary sinus vein being determined from measured current or voltage between the ring-shaped measuring electrode 4 and the counter electrode.

A stimulating and heart signal sensing electrode 10 is positioned in the right atrium and connected by a lead 12 to the heart stimulator 2. A stimulating and heart signal sensing electrode 14 is positioned in the right ventricular and connected to heart stimulator 2 by a lead 16, and a stimulating and heart signal sensing electrode 18 is also inserted into the coronary sinus vein to facilitate contact with the left ventricle. One of these stimulation and sensing electrodes 10, 14, 18 can be used as counter electrode for the blood flow measurements.

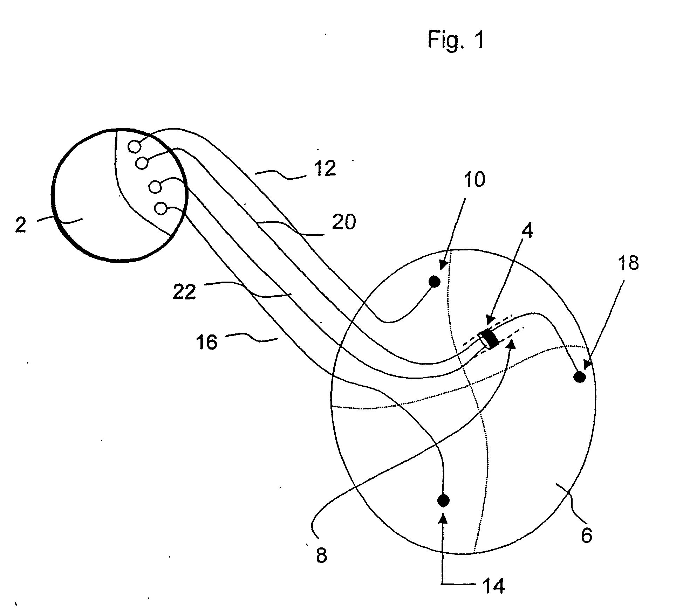

The electrodes shown in figure 1 is just an example of positioning the electrodes of the heart stimulator for multi-site stimulation according to the invention. The number of the electrodes can be reduced or additional electrodes can be positioned in other places of the heart 6.

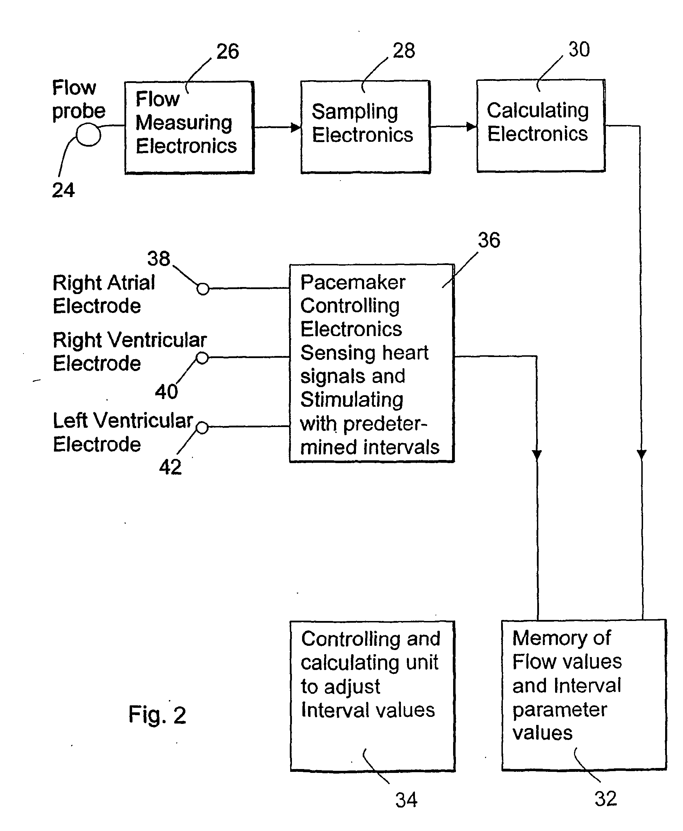

Figure 2 is a block diagram illustrating the electronic circuitry of the heart stimulator according to the invention. The flow measuring electrode 4 in figure 1 is connected to the input 24 in figure 2.

The flow measuring electronics 26 can be of the kind described in US 5,602,342 or that of any other commercially available fluid flow measuring apparatus.

By the sampling electronics 28 a number of blood flow values are recorded with suitable time intervals during the whole or part of the cardiac cycle.

The calculating electronics 30 makes a compilation of the sampled values. The result of this compilation can be minimum flow value, maximum flow value, average flow value, standard deviation of the flow values, etc.

A memory 32 is provided for storing flow values of interest together with heart stimulator parameter values like time intervals. Of primary interest in this connection are average and a minimum perfusion blood flow and time intervals between stimulations at different sites in the heart 6. This interval parameters or delays are either measured values or interval values by controlled by controlling and calculating unit 34.

In the controlling and calculating unit 34 stored values and actually measured flow values related to the actual heart rate are used to select and adjust one or more of the time intervals or delays to be used for control of the delivery of stimulation pulses. The time intervals of interest are for examples AV-delay, right to left ventricle delay or vice-versa.

The pacemaker controlling unit 36 is a conventional pulse generator including means for heart stimulation and heart signal sensing means as well as timing control means for controlling the stimulation. All time intervals, other than the delay intervals discussed above, used in this unit 36 can be programmed from an external programmer and stored, or adjusted from a rate responsive control circuit.

In the embodiment shown in figure 2 the pacemaker controlling electronics 36 comprises three outputs 38, 40, 42 for stimulation and/or sensing in the right atrium, and the right and left ventricles respectively.

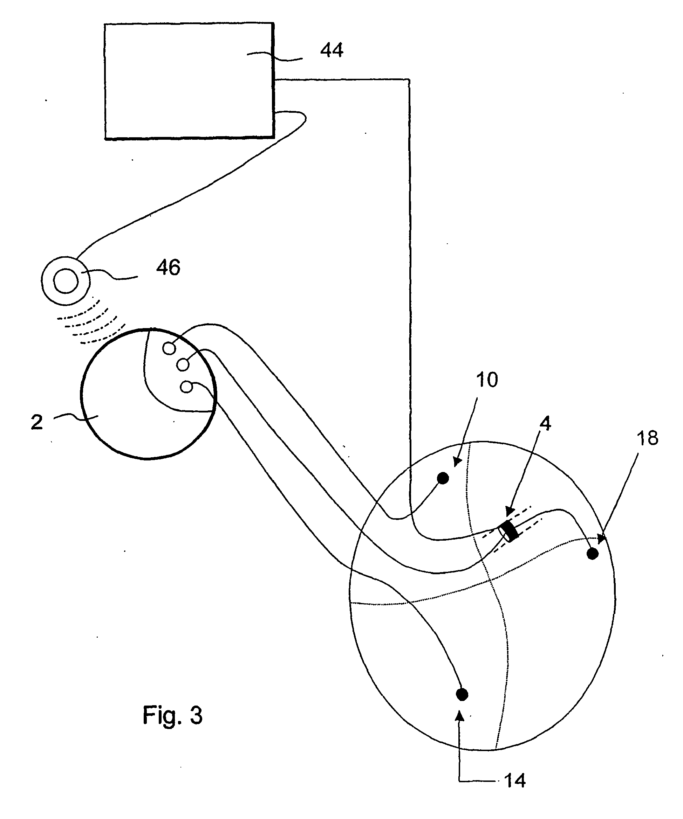

Figure 3 illustrates a modification of the embodiment disclosed in figure 1 and corresponding components and parts of the embodiments of figure 1 and figure 3 are denoted by the same reference numeral. Further, that part of the embodiment in figure 3 which is identical to the embodiment in figure 1 will not be described again but only the differences of the embodiment shown in figure 3 vis-à-vis the embodiment in figure 1.

In the embodiment according to figure 3 the electronic circuitry of the flow measuring device is located in an external unit 44. This unit 44 is capable of communicating with the implanted heart stimulator 2 by ordinary telemetry means 46.

For the blood flow measurements an electrolytic technique as described above can be used, the measuring probe 4 including a ring-shaped measuring electrode 4. The probe 4 can, however, also be a probe designed for Doppler or thermodilution flow measurements. The flow measurements can be performed also by other techniques, e.g. by using ultrasound or NMR.

Measuring, sampling and calculating flow values are performed in the external unit 44 and these flow values are stored also in this external unit 44 together with associated time intervals used by the heart stimulator 2 in operation. These time intervals are delays related to heartbeats and other control intervals, like AV-delays and right to left ventricle delays which are stored together with the heart rate. Heart beat intervals related to stimulations are separated from spontaneous ones.

After the evaluation procedure in the unit 44 compiled or calculated time interval values are transferred by the telemetry channel 46 to the heart stimulator 2 for the control of its subsequent operation.

Principally the heart stimulator according to the invention operates e.g. as follows.

The last measured perfusion blood flow values are stored together with related pulse generator parameter settings. Measured flow values during constant heart rates are compared for different time delay intervals. For each pulse generator parameter setting an average flow values from at least two cardiac cycles is used. The delay intervals are adjusted in that direction, which gives the highest measured flow values. Thus new flow values from the last cardiac cycles are stored and compared to the previous values. If the new result indicates increased blood flow further adjustment of the delay intervals is performed in the same direction as the preceding adjustment. If the result indicates decreased flow the delay interval settings is returned to the preceding one. Alternatively, when a decreased flow is detected the delay interval setting can be adjusted in the direction toward the preceding setting by a smaller step.

The simplest way of adjusting the delay interval setting is to adjust just one parameter at each time and when an optimal setting of this parameter is achieved, a second parameter is adjusted in a corresponding way, etc. As mentioned above the four parameters of interest are normally a) delay time between right and left ventricle for stimulation in each one of these two heart cavities, b) delay time between right and left ventricles when a spontaneous heart beat is starting in one of the chambers, delay times between atrial and ventricle activation, c) when both activations are stimulated, and d) when atrial activation is spontaneous. Additional interval parameter values are of interest if also the left atrium is involved in the sensing and stimulation.

Alternative adjustment procedures are of course possible. Thus another parameter then the latest adjusted one can be adjusted. This will require a more complicated comparison procedure including several comparisons.

As another alternative that parameter is selected from several used parameters for the next adjustment which has given the highest increase in the measured blood flow for the preceding parameter setting.

Also adjustment of two or more parameters at same time is possible. Such multi parameter tuning or controlling of the heart stimulator will more quickly give the best parameter setting.

The heart stimulator according to the invention can also include other control systems for heart rate modulation depending on the blood flow need of the patient's body.

When the heart rate is varying as a result of a rate responsive system of the heart stimulator or as a result of the hearts own natural heart rate control system the parameters of importance for the perfusion blood flow in the heart must be adjusted continuously.

## Claims

1. A heart stimulator for multi-site stimulation of a patient's heart (6) comprising a pulse generator and a measuring device (4, 24, 26, 28,30; 44) for measuring a physiological cardiac parameter and delivering a corresponding output signal to a control unit for controlling time intervals between pulses delivered to different stimulation sites in the heart depending on said output signal according to a predetermined criterion, **characterized in that** said measuring device comprises a ring-shaped electrode (4) which is sized to fit within and is adapted for measuring the blood flow in a coronary sinus vein (8), a counter electrode (10, 14, 18) intended to be brought into contact with the blood, and a measuring unit (26, 44) adapted to measure the voltage or current between said electrodes and to determine therefrom, as said physiological parameter, the perfusion blood flow in said vessel.

2. The heart stimulator according to claim 1, **characterized in that** said measuring device (14, 24, 26, 28, 30; 44) is adapted to measure changes in the perfusion blood flow in response to changes of said time intervals.

3. The heart stimulator according to any one of the preceding claims, **characterized in that** said control unit comprises a memory (32) for storage of latest measured flow values and associated time interval values.

4. The heart stimulator according to claim 3, **characterized in that** said memory (32) is adapted to store measured flow values from at least two consecutive heart cycles with constant parameter settings.

5. The heart stimulator according to claims 3 or 4, **characterized in that** said control unit (32, 34) is adapted to compare measured flow values with stored flow values for constant stimulation rate and adjust said time intervals depending on the result of said comparison.

6. The heart stimulator according to any one of the preceding claims, **characterized in that** said control unit (32, 34) is adapted to adjust one of said time intervals at a time.

7. The heart stimulator according to any one of the claims 1 - 5, **characterized in that** said control unit (32, 34) is adapted for multi-interval adjustment.

8. The heart stimulator according to any one of the preceding claims, **characterized in that** said control unit (32, 34) is adapted to adjust said time intervals in steps of varying sizes.

9. The heart stimulator according to any one of the preceding claims, **characterized in that** said criterion means that the minimum blood flow during a cardiac cycle is increased as much as possible.

10. The heart stimulator according to any one of the claims 1 - 8, **characterized in that** said predetermined criterion means maximising the integral of the blood flow during a cardiac cycle for a certain heart rate.

11. The heart stimulator according to any one of the preceding claims, **characterized in that** said determining means comprises an external determining apparatus (44).

12. The heart stimulator according to claim 11, **characterized in that** telemetry means (46) are provided to transfer said blood flow signal from said external determining apparatus (44) to said control unit.

13. The heart stimulator according to any one of the claims 1 - 11, **characterized in that** said control unit comprises an external control apparatus (44) adapted to receive said blood flow signal and adjust said time intervals according to said predetermined criterion, telemetry means (46) being provided to transfer signals for adjusting said time intervals.

## Patentansprüche

1. Herzstimulator für eine Multisite-Stimulation des Herzens (6) eines Patienten, enthaltend einen Impulsgenerator und eine Messvorrichtung (4, 24, 26, 28, 30; 44) zum Messen eines physiologischen Herzparameters und zum Ausgeben eines entsprechenden Ausgangssignals an eine Steuereinheit zum Steuern von Zeitintervallen zwischen Impulsen, die zu den verschiedenen Stimulationsstellen im Herzen abhängig vom genannten Ausgangssignal entsprechend einem vorbestimmten Kriterium ausgegeben werden, **dadurch gekennzeichnet, dass** die genannte Messvorrichtung eine ringförmige Elektrode (4) enthält, die so bemessen ist, dass sie in eine koronare Sinusvene (8) passt und ausgelegt ist, den Blutfluss in dieser Vene zu messen, ferner eine Gegenelektrode (10, 14, 18), die mit dem Blut in Kontakt zu bringen ist, sowie eine Messeinheit (26, 44), die ausgelegt ist, die Spannung oder den Strom zwischen den genannten Elektroden zu messen und hieraus als den genannten physiologischen Parameter den Perfusionsblut-fluss in dem genannten Gefäß zu bestimmen.

2. Herzstimulator nach Anspruch 1, **dadurch gekennzeichnet, dass** die genannte Messvorrichtung (14, 24, 26, 28, 30; 44) ausgelegt ist, Änderungen im Perfusionsblutfluss in Reaktion auf Änderungen der genannten Zeitintervalle zu messen.

3. Herzstimulator nach einem der vorhergehenden Ansprüche, **dadurch gekennzeichnet, dass** die genannte Steuereinheit einen Speicher (32) zum Speichern der zuletzt gemessenen Flusswerte und der zugehörigen Zeitintervallwerte enthält.

4. Herzstimulator nach Anspruch 3, **dadurch gekennzeichnet, dass** der genannte Speicher (32) ausgelegt ist, gemessene Flusswerte von wenigstens zwei aufeinanderfolgenden Herzzyklen mit konstanten Parametereinstellungen zu speichern.

5. Herzstimulator nach Anspruch 3 oder 4, **dadurch gekennzeichnet, dass** die genannte Steuereinheit (32, 34) ausgelegt ist, gemessene Flusswerte mit gespeicherten Flusswerten für eine konstante Stimulationsfrequenz zu vergleichen und die genannten Zeitintervalle abhängig von dem Ergebnis des genannten Vergleichs einzustellen.

6. Herzstimulator nach einem der vorhergehenden Ansprüche, **dadurch gekennzeichnet, dass** die genannte Steuereinheit (32, 34) ausgelegt ist, jeweils einen der genannten Zeitintervalle einzustellen.

7. Herzstimulator nach einem der Ansprüche 1 bis 5, **dadurch gekennzeichnet, dass** die genannte Steuereinheit (32, 34) für eine Mehrfachintervalleinstellung ausgelegt ist.

8. Herzstimulator nach einem der vorhergehenden Ansprüche, **dadurch gekennzeichnet, dass** die genannte Steuereinheit (32, 34) ausgelegt ist, die genannten Zeitintervalle in Schritten von variierenden Größen einzustellen.

9. Herzstimulator nach einem der vorhergehenden Ansprüche, **dadurch gekennzeichnet, dass** das genannte Kriterium bedeutet, dass der minimale Blutfluss während eines Herzzyklus so viel wie möglich erhöht wird.

10. Herzstimulator nach einem der Ansprüche 1 bis 8, **dadurch gekennzeichnet, dass** das genannte vorbestimmte Kriterium bedeutet, das Integral des Blutflusses während eines Herzzyklus für eine bestimmte Herzfrequenz zu maximieren.

11. Herzstimulator nach einem der vorhergehenden Ansprüche, **dadurch gekennzeichnet, dass** die genannte Bestimmungsvorrichtung ein externes Bestimmungsgerät (44) enthält.

12. Herzstimulator nach Anspruch 11, **dadurch gekennzeichnet, dass** eine Telemetrievorrichtung (46) vorgesehen ist, um das genannte Blutflusssignal aus dem genannten externen Bestimmungsgerät (44) zu der genannten Steuereinheit zu übertragen.

13. Herzstimulator nach einem der Ansprüche 1 bis 11, **dadurch gekennzeichnet, dass** die Steuereinheit ein externes Steuergerät (44) enthält, das ausgelegt ist, das genannte Blutflusssignal zu empfangen und die genannten Zeitintervalle gemäß dem genannten vorbestimmten Kriterium einzustellen, ferner eine Telemetrievorrichtung (46) vorgesehen ist, um Signale zum Einstellen der genannten Zeitintervalle zu übertragen.

## Revendications

1. Stimulateur cardiaque pour une stimulation multisite d'un coeur ( 6 ) de patient, comprenant un générateur d'impulsions et un dispositif ( 4, 24, 26, 28, 30 ; 44 ) de mesure d'un paramètre cardiaque physiologique et d'envoi d'un signal de sortie correspondant à une unité de commande des intervalles de temps entre des impulsions envoyées à des sites de stimulation différents du coeur en fonction du signal de sortie suivant un critère déterminé à l'avance,
**caractérisé en ce que** le dispositif de mesure comprend une électrode ( 4 ) de forme annulaire, qui est dimensionnée pour s'adapter dans une veine ( 8 ) sinus coronaire et est conçue pour y mesurer le flux sanguin, une contre-électrode ( 10, 14, 18 ) destinée à être mise en contact avec le sang, et une unité ( 26, 44 ) de mesure conçue pour mesurer la tension ou le courant entre les électrodes et pour en déterminer, en tant que paramètre physiologique, le flux sanguin d'irrigation du vaisseau.

2. Stimulateur cardiaque suivant la revendication 1, **caractérisé en ce que** le dispositif ( 14, 24, 26, 28, 30 ; 44 ) de mesure est conçu pour mesurer des variations du flux sanguin d'irrigation en réponse à des variations des intervalles de temps.

3. Stimulateur cardiaque suivant l'une quelconque des revendications précédentes, **caractérisé en ce que** l'unité de commande comprend une mémoire ( 32 ) de mémorisation des dernières valeurs de flux mesurées et des valeurs associées des intervalles de temps.

4. Stimulateur cardiaque suivant la revendication 3, **caractérisé en ce que** la mémoire ( 32 ) est conçue pour mémoriser des valeurs de débit mesurées d'au moins deux cycles cardiaques consécutifs ayant des réglages de paramètre constants.

5. Stimulateur cardiaque suivant les revendications 3 ou 4, **caractérisé en ce que** l'unité ( 32, 34 ) de commande est conçue pour comparer les valeurs de débit mesurées à des valeurs de débit mémorisées pour un rythme de stimulation constant et pour régler les intervalles de temps en fonction du résultat de la comparaison.

6. Stimulateur cardiaque suivant l'une quelconque des revendications précédentes, **caractérisé en ce que** l'unité ( 32, 34 ) de commande est conçue pour régler au moins l'un des intervalles de temps à la fois.

7. Stimulateur cardiaque suivant l'une quelconque des revendications 1 à 5, **caractérisé en ce que** l'unité ( 32, 34 ) de commande est conçue pour un réglage multi-intervalles.

8. Stimulateur cardiaque suivant l'une quelconque des revendications précédentes, **caractérisé en ce que** l'unité ( 32, 34 ) de commande est conçue pour régler les intervalles de temps en stades de dimensions variables.

9. Stimulateur cardiaque suivant l'une quelconque des revendications précédentes, **caractérisé en ce que** le critère signifie que le débit sanguin minimum pendant un cycle cardiaque est augmenté autant que possible.

10. Stimulateur cardiaque suivant l'une quelconque des revendications 1 à 8, **caractérisé en ce que** le critère déterminé à l'avance signifie de rendre maximum l'intégrale du débit sanguin pendant un cycle cardiaque pendant un certain rythme cardiaque.

11. Stimulateur cardiaque suivant l'une quelconque des revendications précédentes, **caractérisé en ce que** les moyens de détermination comprennent un appareil ( 44 ) extérieur de détermination.

12. Stimulateur cardiaque suivant la revendication 11, **caractérisé en ce qu'**il est prévu des moyens ( 46 ) de télémétrie pour transférer le signal de débit sanguin de l'appareil ( 44 ) extérieur de détermination à l'unité de commande.

13. Stimulateur cardiaque suivant l'une quelconque des revendications 1 à 11, **caractérisé en ce que** l'unité de commande comprend un dispositif ( 44 ) extérieur de commande conçu pour recevoir le signal de débit sanguin et pour régler les intervalles de temps en fonction du critère déterminé à l'avance, des moyens ( 46 ) de télémétrie étant prévus pour transférer des signaux pour régler les intervalles de temps.
